# EUROPEAN PATENT SPECIFICATION

(11) **EP 2 206 694 B1**
(45) Date of publication and mention of the grant of the patent: **12.02.2014**
(21) Application number: 08792582.2
(22) Date of filing: 21.08.2008
(51) Int. Cl.: C07C 29/16, C07C 31/125, C07C 31/135, C07C 31/27, C07B 61/00

(54) **METHOD FOR PRODUCING ALCOHOL USING CARBON DIOXIDE AS RAW MATERIAL**
VERFAHREN ZUR HERSTELLUNG VON ALKOHOL UNTER VERWENDUNG VON KOHLENDIOXID ALS ROHMATERIAL
PROCÉDÉ DE PRODUCTION D'ALCOOL À L'AIDE DE DIOXYDE DE CARBONE COMME MATIÈRE PREMIÈRE

(30) Priority: 25.09.2007 JP 2007246792
(43) Date of publication of application: 14.07.2010
(73) Proprietor: Hitachi Chemical Company, Ltd., Chiyoda-ku Tokyo 100-6606 (JP); National Institute of Advanced Industrial Science and Technology, Tokyo 100-8921 (JP)
(72) Inventor: KAMEI, Junichi, Ichihara-shi Chiba 290-8567 (JP); KIKUCHI, Tooru, Hitachi-shi Ibaraki 317-8555 (JP); TOMINAGA, Kenichi, Tsukuba-shi Ibaraki 305-8565 (JP); SATO, Kazuhiko, Tsukuba-shi Ibaraki 305-8565 (JP)
(74) Representative: HOFFMANN EITLE
(86) International application number: PCT/JP2008/064861
(87) International publication number: WO 2009/041192

(56) References cited:
- WO-A1-2007/111091
- JP-A- 55 118 429
- JP-A- 2001 233 795
- JAASKELAINEN, S ET AL.: 'The use of carbon dioxide in ruthenium carbonyl catalyzed 1-hexene hydroformylation promoted by alkali metal and alkaline earth salts' APPLIED CATALYSIS A:GENERAL vol. 247, 2003, pages 95 - 100, XP004434429
- TOMINAGA, K ET AL.: 'Ruthenium-catalyzed one-pot hydroformylation of alkenes using carbon dioxide as a reactant' JOURNAL OF MOLECULAR CATALYSIS A:CHEMICAL vol. 220, 2004, pages 159 - 165, XP008132217

## Description

### TECHNICAL FIELD

The invention relates to a method for producing an alcohol, in particular, the invention relates to a method for producing an alcohol by hydroformylation using carbon dioxide as a raw material.

### BACKGROUND ART

In recent years, as a common method for producing an alcohol, a method is known in which a raw material organic compound having an unsaturated carbon-carbon bond is hydrated or hydroformylated. In the former method, formation of a secondary or tertiary alcohol preferentially occurs according to the markownikoff rule. Therefore, in order to produce a primary alcohol, it is necessary to use a hydroformylation reaction. For this reason, a hydroformylation reaction has become one of the most important processes in the chemical industry, and is used for the production of 6,000,000 tons or more of chemical products on an annual basis in the world.

However, in the hydroformylation reaction, a large amount of carbon monoxide, which is significantly toxic, is used as a raw material. Therefore, in order to perform this reaction on the industrial basis, a large amount of investment is needed for safety control and preservation of the environment.

As the method for solving this problem, the inventors have developed a novel hydroformylation method in which a ruthenium compound is used as a catalyst, by which carbon dioxide, which is much safer to both human bodies and the environment, as compared with carbon monoxide, can be used as a raw material (Patent Document 1). Further, the inventors have developed a method for producing an alcohol by selectively hydroformylating a common raw material compound with carbon dioxide, by using as a catalyst a dispersion obtained by dispersing a ruthenium compound in a non-aqueous ionic liquid composed of an organic or inorganic salt which is liquefied at around room temperature (Patent Document 2). In addition, as stated in Japanese Patent Application No. 2006-087788, the inventors developed a method in which a reaction speed and a yield have remarkably improved by using an acid in combination in the above-mentioned production method.

In the conventional hydroformylation reaction see e.g. EP2000453 in which carbon dioxide is used as a raw material, it was indispensable to use a ruthenium-containing compound as a catalyst in order to activate carbon dioxide. Generally, ruthenium is present in a mineral containing platinum, and can be produced in combination with platinum. The production amount thereof is much smaller than that of platinum, and hence it is a very rare metal. While the amount of platinum in the crust is 0.01 ppm, the amount of ruthenium is only 0.0004 ppm which accounts for 4% of the amount of platinum (The Merck Index 12^{th} Edition). In the past, since the demand for ruthenium was small, the price thereof was lowest among the metals of the platinum group. However, in recent years, it has come to be used in electronics materials including chip registers, plasma display panels and hard disks, resulting in a steep rise in the price thereof (Report by Johnson Matthey Plc; Platinum 2007).
Patent Document 1: JP-A-2001-233795
Patent Document 2: JP-A-2004-091331

### DISCLOSURE OF THE INVENTION

The object of the invention is to provide a method for producing an alcohol efficiently from an organic compound containing an unsaturated carbon-carbon bond, carbon dioxide and hydrogen by using a small amount of ruthenium.

As a result of intensive studies, the inventors have found a method which is capable of producing an intended alcohol in a high yield using ruthenium in an amount smaller than the conventional amount by using a catalyst system comprising a ruthenium compound and a cobalt compound.

According to the invention, the following method for producing an alcohol can be provided.
1. A method for producing an alcohol comprising hydroformylating an organic compound having an unsaturated carbon-carbon bond which is selected from the group consisting of aliphatic linear unsaturated compounds, aliphatic cyclic unsaturated compounds, aromatic chain-like unsaturated compounds and aromatic cyclic unsaturated compounds with carbon dioxide and hydrogen by using a catalyst system comprising a ruthenium compound and a cobalt compound, wherein the hydroformylation is conducted at a temperature of 100°C to 200°C at a pressure of 1 to 50 MPa.
2. The method for producing an alcohol according to 1, wherein the catalyst system further comprises a halide salt.
3. The method for producing an alcohol according to 1 or 2, wherein the catalyst system further comprises an acid.
4. The method for producing an alcohol according to 3, wherein the acid is a Bronsted acid.
5. The method for producing an alcohol according to 4, wherein the Bronsted acid is an acid containing phosphor.
6. The method for producing an alcohol according to any of 1 to 5, wherein the organic compound has an unsaturated carbon-carbon bond not at the terminal of its molecule but inside the molecule.
7. The method for producing an alcohol according to any of 1 to 6, wherein the organic compound is a compound having two or more unsaturated carbon-carbon bonds and a polyvalent alcohol is produced using this organic compound.

According to the invention, it is possible to provide a method which is capable of producing efficiently from an organic compound having an unsaturated carbon-carbon bond, carbon dioxide and hydrogen as a raw material using a small amount of ruthenium.

### BEST MODE FOR CARRYING OUT THE INVENTION

In the method for producing an alcohol of the invention, an organic compound having an unsaturated carbon-carbon bond is hydroformylated with carbon dioxide and hydrogen, and a catalyst system comprising a ruthenium compound and a cobalt compound is used in this hydroformylation. This catalyst system can further comprise a halide salt and/or an acid.

The organic compound having an unsaturated carbon-carbon bond used in the invention is selected from the group consisting of aliphatic linear unsaturated compounds, aliphatic cyclic unsaturated compounds, aromatic chain-like unsaturated compounds and aromatic cyclic unsaturated compounds. The unsaturated carbon-carbon bond may be present either at the terminal of its molecule or inside the molecule. Also, an organic compound having a plurality of unsaturated carbon-carbon bonds may also be used. By using as a raw material an organic compound having a plurality of unsaturated carbon-carbon bonds, it is possible to produce a polyvalent alcohol having a plurality of hydroxymethyl groups (-CH₂OH). In these organic compounds, the hydrogen atom in the molecule may be substituted with at least one group selected from an alkyl group, a cyclic aliphatic group, an aromatic group, a heterocyclic group, a carbonyl group, an alkoxy group, a cyano group, an amino group, an amide group, a nitro group, a halogen and a phosphor-containing substituent.

Examples of the aliphatic chain-like unsaturated compounds include ethylene, propylene, butylene, pentene, hexene, heptene, octene, nonene, decene, undecene, dodecene, tridecene, tetradecene, pentadecene, hexadecene, heptadecene, octadecene, nonadecene, butadiene, pentadiene, hexadiene, heptadiene, octadiene, nonadiene, hexanetriene, heptatriene, octatriene, and isomers, derivatives or the like thereof. Examples of the aliphatic cyclic unsaturated compounds include, for example, cyclopentene, cyclohexene, cycloheptene, cyclooctene, cyclopentadiene, cyclohexadiene, cycloheptadiene, cyclooctadiene, tetrahydroindene, methyltetrahydroindene, norbornene, norbornadiene, methylvinylnorbornene, dicyclopentadiene, methyldicyclopentadiene, tricyclopentadiene, tetracyclopentadiene, and isomers, derivatives or the like thereof. Examples of the aromatic chain-like unsaturated compounds include styrene, stilbene, triphenylethylene, tetraphenylethylene, and derivatives thereof or the like. Examples of the aromatic cyclic unsaturated compounds include indene, dihydronaphthalene, indole, and derivatives thereof or the like.

In the hydroformylation reaction of the invention, hydrogen and carbon dioxide are used. They may be supplied in the form of a mixed gas or may be supplied separately. The mixed gas is a mixed gas (raw material gas) composed mainly of hydrogen and carbon dioxide. The mixed gas contains carbon dioxide preferably in an amount of 10 to 95 vol%, more preferably 50 to 80 vol%, and contains hydrogen preferably in an amount of 5 to 90 vol%, more preferably 20 to 50 vol%. If the content of hydrogen exceeds 90%, hydrogenation of the raw material may occur dominantly. If the content of hydrogen is less than 5%, the reaction speed may significantly be lowered. Carbon monoxide is not necessarily mixed in the raw material gas, but it may be mixed in.

There are no specific restrictions on the ruthenium compound used in the invention insofar as it contains ruthenium. Preferred examples include ruthenium compounds having a carbonyl ligand such as Ru₂(CO)₆Cl₄, Ru₃(CO)₁₂, H₄Ru₄(CO)₁₂, H₂Ru₆(CO)₁₈, and H₂Ru₆C(CO)₁₆. Further, these ruthenium compounds having a carbonyl ligand can be obtained by subjecting to a carbonylation method ruthenium compounds as a raw material such as RuCl₃, RuCl₂(C₈H₁₂), Ru(CO)₃(C₈H₈). Ru(CO)₃(C₈H₁₂), and Ru(C₈H₁₀)(C₈H₁₂) before or during the reaction. As for the common carbonylation reaction, a method in which carbon monoxide is added and heated, or a method in which formic acid is added and heated or the like are known.

The ruthenium compound is used preferably in an amount of 1/10000 to 1 equivalent, more preferably 1/1000 to 1/50 equivalent relative to the raw material compound. If the amount of the ruthenium compound is too small, the reaction speed tends to be slow. If the amount of the ruthenium compound is too large, the raw material compound may be hydrogenated before the reaction of carbon dioxide.

There are no specific restrictions on the cobalt compound used in the invention insofar as it contains cobalt. Preferred examples include cobalt compounds containing a carbonyl ligand such as CO₂(CO)₈ and HCO(CO)₄, or cobalt compounds containing a carboxylic acid as a ligand such as cobalt acetate, cobalt propionate, cobalt benzoate and cobalt citrate.

The amount of the cobalt compound used is preferably 1/100 to 10 equivalent, more preferably 1/10 to 5 equivalent. If the amount ratio is lower than 1/100, effects of accelerating hydroformylation are low. If the amount ratio of the cobalt compound is higher than 10, hydrogenation of a raw material, which is a side reaction, may be easily accelerated.

In the catalyst system of the invention, a halide salt is preferably added. As the cation used in the halide salt, either inorganic ions or organic ions may be used.

Preferred examples of the halide salt include chloride salts, bromide salts and iodide salts.

The added amount of the halide salt is, for example, 1 to 1000 equivalent, preferably 2 to 100 equivalent relative to the added amount of the ruthenium compound. If the added amount of the halide salt is less than 1 equivalent, the reaction speed may be slow. If the amount of the halide salt exceeds 1000 equivalents, no further effects of acceleration of the reaction can be obtained.

Examples of the inorganic ions used in the halide salt include, for example, lithium, sodium, potassium, rubidium, cesium, calcium and strontium. Examples of the organic ions include, for example, tetramethyl ammonium, tetraethyl ammonium, tetrapropyl ammonium, tetrabutyl ammonium, tetrapentyl ammonium, tetrahexyl ammonium, tetraheptyl ammonium, tetraoctyl ammonium, ethyltrimethyl ammonium, propyltrimethyl ammonium, butyltrimethyl ammonium, hexyltrimethyl ammonium, octyltrimethyl ammonium, decyltrimethyl ammonium, dodecyltrimethyl ammonium, tetradecyltrimethyl ammonium, hexadecyltrimethyl ammonium, octadecyltrimethyl ammonium, benzyltrimethyl ammonium, benzyltriethyl ammonium, benzyltributyl ammonium, tetramethyl phosphonium, tetraethyl phosphonium, tetraphenyl phosphonium, benzytriphenyl phosphonium and bis(triphenylphosphine)iminium.

The halide salt is not necessarily a solid salt. Preferably, the halide salt is a non-aqueous ionic liquid containing a halide ion which is liquefied at around room temperature or in a temperature range of 100°C or lower.

Examples of the cation used as the non-aqueous ionic liquid include
1-ethyl-3-methyl-imidazolium, 1-propyl-3-methylimidazolium,
1-butyl-3-methylimidazolium, 1-pentyl-3-methylimidazolium,
1-hexyl-3-methylimidazolium, 1-heptyl-3-methylimidazolium,
1-octyl-3-methylimidazolium, 1-decyl-3-methylimidazolium,
1-dodecyl-3-methylimidazolium, 1-tetradecyl-3-methylimidazolium,
1-hexadecyl-3-methylimidazolium, 1-octadecyl-3-methylimidazolium,
1-ethyl-2,3-dimethylimidazolium, 1-butyl-2,3-dimethylimidazolium,
1-hexyl-2,3-dimethylimidazolium, 1-ethylpyridinium, 1-butylpydinium, 1-hexylpyridinium,
8-methyl-1,8-diazabicyclo[5.4.0]-7-undecene,
8-ethyl-1,8-diazabicyclo[5.4.0]-7-undecene,
8-propyl-1,8-diazabicyclo[5.4.0]-7-undecene,
8-butyl-1,8-diazabicyclo[5.4.0]-7-undecene,
8-pentyl-1,8-diazabicyclo[5.4.0]-7-undecene,
8-hexyl-1,8-diazabicyclo[5.4.0]-7-undecene,
8-heptyl-1,8-diazabicyclo[5.4.0]-7-undecene and
8-octyl-1,8-diazabicyclo[5.4.0]-7-undecene. These halide salts may be used either singly or in combination.

An acid may be added to the catalyst system used in the invention. Any acid may be used insofar as it is an acid which can be applied to the definition of a Lewis acid. According to this definition, when a certain material A accepts a pair of electrons from another material B, the material A is defined as an acid and the material B is defined as a base. In the invention, all acids which can be applied to the material A which accept electron pairs can be used.

As the above-mentioned acid, A is preferably an acid which supplies a proton, that is, a Bronsted acid. Examples of the Bronsted acid include, for example, hydrochloric acid, sulfuric acid, nitric acid, phosphoric acid, methylphosphoric acid, alkylphosphoric acid, phenylphosphoric acid, phenylphosphonic acid, phenylphosphinic acid, boric acid, phenylboric acid, trifluoromethanesulfonic acid, paratoluenesulfonic acid, phenol, tungstic acid, phosphotungstic acid, an alkylcarboxylic acid represented by formic acid, acetic acid, trifluoroacetic acid, propionic acid and lactic acid, and aromatic carboxylic acids represented by benzoic acid, phthalic acid and salicylic acid. Preferable acids are an acid containing phosphor such as phosphoric acid, alkylphosphoric acid and phenylphosphoric acid.

The added amount of an acid is 0.1 to 100 equivalent, preferably 1 to 10 equivalent, relative to the amount of the ruthenium compound. If the added amount of an acid is less than 0.1 equivalent relative to the amount of the ruthenium compound, almost no effects of accelerating the reaction by the addition of an acid can be found. If the added amount of an acid exceeds 100 equivalent, productivity may be lowered significantly.

The hydroformylation reaction is conducted in a temperature range of 100 to 200°C, more preferably 120 to 180°C. In a temperature range lower than 100°C, carbon dioxide may not be reacted. In a temperature range higher than 200°C, hydrogenation of an unsaturated bond may occur preferentially.

The hydroformylation reaction occurs at a pressure of 1 to 50 MPa, more preferably 2 to 15 MPa. If the pressure is lower than 1 MPa, the reaction may be slow. If the pressure exceeds 50 MPa, no further effects of accelerating the reaction can be obtained.

In the production method according to the invention, a solvent may be used in a reaction system, for example. There are no specific restrictions on the solvent insofar as they can dissolve a raw material for the reaction. Preferred examples of the solvent include n-pentane, n-hexane, n-heptane, cyclohexane, benzene, toluene, o-xylene, p-xylene, m-xylene, ethylbenzene, cumene, tetrahydrofuran, N-methylpyrrolidone, dimethylformamide, dimethylacetoamide, dimethylimidazolidinone, ethylene glycol dimethyl ether, diethylene glycol dimethyl ether and triethylene glycol dimethyl ether. When a solvent is used, it is preferred that the solvent be used in such an amount that the concentration of the raw material compound becomes 0.1 vol% or more, preferably 1.0 vol% or more.

### [Examples]

The inventors have already developed a method for producing an alcohol using a catalyst system composed of a ruthenium compound and an acid, and applied a patent for this invention (Patent Application No. 2006-087788). The present invention is a further modification of the above-mentioned invention, in which the amount of the ruthenium compound is reduced. The following comparative examples 2 and 3 correspond to the invention of the prior application.

### Example 1

### [Hydroformylation of cyclohexene using a catalyst system composed of a ruthenium compound, a cobalt compound and a chloride salt]

At room temperature, a stainless steel-made pressurized reactor having an inner volume of 50 ml was charged with 0.05 mmol of Ru₂(CO)₆Cl₄ as a ruthenium compound, 0.05 mmol of CO₂(CO)₈ as a cobalt compound, 2.5 mmol of hexadecyltrimethylammonium chloride as a halide salt, 10.0 mmol of cyclohexene as a raw material organic compound and 10.0 mL of toluene as a solvent, followed by stirring to cause them to be dissolved. Then, carbon dioxide and hydrogen were introduced such that the partial pressure each of these gases became 4 MPa, and the resultant was retained at 140°C for 15 hours. Thereafter, the reactor was cooled to room temperature, and the remaining organic phase was extracted by releasing pressure. The extracted organic phase was examined by gas chromatography. The conversion ratio of cyclohexene was 54%. As hydroformylated products, cyclohexane carboxaldehyde was formed in a yield of 20%, cyclohexane methanol was formed in a yield of 20%, and cyclohexane methanol was formed in a yield of 24%. Cyclohexane was formed as a hydrogenated product in a yield of 2%.

### Example 2

### [Hydroformylation of cyclohexene using a catalyst system composed of a ruthenium compound, a cobalt compound, a chloride salt and phenylphosphoric acid]

A reaction was conducted in the same manner as in Example 1, except that phenylphosphoric acid was added in an amount of 0.25 mmol. The conversion ratio of cyclohexene was 86%, and cyclohexane carboxaldehyde was formed in a yield of 1% and cyclohexane methanol was formed in a yield of 71 %, and cyclohexane was formed as a hydrogenated product in a yield of 2%.

### Example 3

### [Hydroformylation of cyclohexene using a catalyst system composed of a ruthenium compound, a cobalt compound, a chloride salt and benzoic acid]

A reaction was conducted in the same manner as in Example 2, except that benzoic acid was used instead of phenylphosphoric acid. The conversion ratio of cyclohexene was 57%, and cyclohexane carboxaldehyde was formed as a hydrogenated product in a yield of 17% and cyclohexane methanol was formed in a yield of 28%, and cyclohexane was formed as a hydrogenated product in a yield of 2%.

### Example 4

### [Hydroformylation of cyclohexene using a catalyst system composed of a ruthenium compound, a cobalt compound, a bromide salt and phenylphosphoric acid]

A reaction was conducted in the same manner as in Example 2, except that hexadecyltrimethylammonium bromide was used as the halide salt instead of hexadecyltrimethylammonium chloride.

The conversion ratio of cyclohexene was 48%, and cyclohexane carboxaldehyde was formed in a yield of 2% and cyclohexane methanol was formed in a yield of 30%, and cyclohexane was formed as a hydrogenated product in a yield of 1%.

### Comparative Example 1

### [Hydroformylation of cyclohexene using a catalyst system composed of a ruthenium compound and a chloride salt]

A reaction was conducted in the same manner as in Example 1, except that Ru₃(CO)₁₂ was used as the ruthenium compound and a cobalt compound was not added. The conversion ratio of cyclohexene was 73%, and cyclohexane carboxaldehyde was formed in a yield of 14% and cyclohexane methanol was formed in a yield of 44%, and cyclohexane was formed as a hydrogenated product in a yield of 3%.

### Comparative Example 2

### [Hydroformylation of cyclohexene using a catalyst system composed of a ruthenium compound, a chloride salt and phenylphosphoric acid]

A reaction was conducted in the same manner as in Example 2, except that Ru₃(CO)₁₂ was used as the ruthenium compound and a cobalt compound was not added. The conversion ratio of cyclohexene was 82%, and cyclohexane carboxaldehyde was formed in a yield of 2% and cyclohexane methanol was formed in a yield of 60%, and cyclohexane was formed as a hydrogenated product in a yield of 2%.

From the above-mentioned results, it can be understood that the reaction proceeds even though ruthenium is used in an amount smaller than the conventional amount by substituting part of a ruthenium compound in the catalyst system with a cobalt compound, and that a high alcohol yield can be obtained with a smaller ruthenium amount by adding an acid. Further, it can be understood that, among acids, a phosphor-containing acid is particularly effective.

### Example 5

### [Hydroformylation of 1-hexene using a catalyst system composed of a ruthenium compound, a cobalt compound, a chloride salt and phenylphosphoric acid]

A reaction was conducted in the same manner as in Example 2, except that 1-hexene was used as the raw material organic compound. As a result, it was found that the conversion ratio was 91 %, and heptanal was formed in a yield of 1% and heptanol was formed in a yield of 68%, and hexane was formed as a hydrogenated product in a yield of 8%.

### Comparative Example 3

### [Hydroformylation of 1-hexene using a catalyst system composed of a ruthenium compound, a chloride salt and phenylphosphoric acid]

A reaction was conducted in the same manner as in Comparative Example 2, except that 1-hexene was used as the raw material organic compound. As a result, it was found that the conversion ratio was 90%, and heptanal was formed in a yield of 1% and heptanol was formed as a hydrogenated product in a yield of 64%, and hexane was formed in a yield of 7%.

From the above-mentioned results, it can be understood that, by substituting part of a ruthenium compound as the catalyst with a cobalt compound, an intended alcohol can be obtained in a high yield with a smaller amount of ruthenium irrespective of the structure, i.e. cyclic or chain-like, of the raw material organic compound.

### Example 6

### [Hydroformylation of cyclohexene using a catalyst system composed of a ruthenium compound, a cobalt compound, a chloride salt as a non-aqueous ionic liquid and benzoic acid]

A reaction was conducted in the same manner as in Example 3, except that 2.5 mmol of 1-butyl-3-methylimidazolyl chloride, which is a non-aqueous inonic liquid, was used as the halide salt. As a result, it was found that the conversion ratio of cyclohexene was 36%, cyclohexane carboaldehyde was formed in a yield of 4%, cyclohexane methanol was formed in a yield of 25% and cyclohexane was formed as a hydrogenated product in a yield of 2%.

From the above-mentioned result, it can be understood that an intended alcohol can be obtained by using a non-aqueous ionic liquid as the halide salt.

### Example 7

### [Production of a polyvalent alcohol using a catalyst system composed of a ruthenium compound, a cobalt compound, a chloride salt and diphenyl phosphite]

A reaction was conducted in the same manner as in Example 2, except that 5.0 mmol of dicyclopentadiene was used as the raw material organic compound, diphenyl phosphite was used instead of phenylphosphoric acid as the acid, and the reaction was conducted at 140°C for 5 hours, and then at 160°C for 10 hours. As a result, it was found that the conversion ratio of dicyclopentadiene was 100%, dicyclopentadiene methanol was formed in a yield of 71 %, dihydrodicyclopentadiene dimethanol was formed in a yield of 17% and dihydrocyclopentadiene was formed as a hydrogenated product in a yield of 5%.

### INDUSTRIAL APPLICABILITY

By the method of the invention, an alcohol can be obtained in a high yield with a small amount of ruthenium. The method of the invention will serve as an aid to put into a practical use an environmentally conscious, inexpensive synthesis of an alcohol which uses, instead of carbon monoxide, carbon dioxide which is safer and less expensive.

## Claims

1. A method for producing an alcohol comprising hydroformylating an organic compound having an unsaturated carbon-carbon bond which is selected from the group consisting of aliphatic linear unsaturated compounds, aliphatic cyclic unsaturated compounds, aromatic chain-like unsaturated compounds and aromatic cyclic unsaturated compounds with carbon dioxide and hydrogen by using a catalyst system comprising a ruthenium compound and a cobalt compound, wherein the hydroformylation is conducted at a temperature of 100°C to 200°C at a pressure of 1 to 50 MPa.

2. The method for producing an alcohol according to claim 1, wherein the catalyst system further comprises a halide salt.

3. The method for producing an alcohol according to claim 1 or 2, wherein the catalyst system further comprises an acid.

4. The method for producing an alcohol according to claim 3, wherein the acid is a Bronsted acid.

5. The method for producing an alcohol according to claim 4, wherein the Bronsted acid is an acid containing phosphor.

6. The method for producing an alcohol according to any of claims 1 to 5, wherein the organic compound has an unsaturated carbon-carbon bond not at the terminal of its molecule but inside the molecule.

7. The method for producing an alcohol according to any of claims 1 to 6, wherein the organic compound is a compound having two or more unsaturated carbon-carbon bonds and a polyvalent alcohol is produced using this organic compound.

## Patentansprüche

1. Verfahren zum Herstellen eines Alkohols, umfassend das Hydroformylieren einer organischen Verbindung, die eine ungesättigte Kohlenstoff-Kohlenstoff-Bindung aufweist, die aus der Gruppe ausgewählt ist, die aus aliphatischen linearen ungesättigten Verbindungen, aliphatischen zyklischen ungesättigten Verbindungen, aromatischen kettenartigen ungesättigten Verbindungen und aromatischen zyklischen ungesättigten Verbindungen besteht, mit Kohlendioxid und Wasserstoff durch Verwenden eines Katalysatorsystems, das eine Rutheniumverbindung und eine Kobaltverbindung umfasst, wobei die Hydroformylierung bei einer Temperatur von 100°C bis 200°C unter einem Druck von 1 bis 50 MPa durchgeführt wird.

2. Verfahren zum Herstellen eines Alkohols gemäß Anspruch 1, worin das Katalysatorsystem ferner ein Halogenidsalz umfasst.

3. Verfahren zum Herstellen eines Alkohols gemäß Anspruch 1 oder 2, worin das Katalysatorsystem ferner eine Säure umfasst.

4. Verfahren zum Herstellen eines Alkohols gemäß Anspruch 3, worin die Säure eine Brönstedsäure ist.

5. Verfahren zum Herstellen eines Alkohols gemäß Anspruch 4, worin die Brönstedsäure eine Phosphor enthaltende Säure ist.

6. Verfahren zum Herstellen eines Alkohols gemäß einem der Ansprüche 1 bis 5, worin die organische Verbindung eine ungesättigte Kohlenstof-Kohlenstoff-Bindung nicht am Ende des Moleküls sondern innerhalb des Moleküls aufweist.

7. Verfahren zum Herstellen eines Alkohols gemäß einem der Ansprüche 1 bis 6, worin die organische Verbindung eine Verbindung ist, die zwei oder mehr ungesättigte Kohlenstoff-Kohlenstoff-Bindungen aufweist, und ein mehrwertiger Alkohol durch Verwenden dieser organischen Verbindung hergestellt wird.

## Revendications

1. Procédé de production d'alcool comprenant l'hydroformylation d'un composé organique comportant une liaison carbone-carbone insaturée qui est choisi dans le groupe constitué des composés insaturés linéaires aliphatiques, des composés insaturés cycliques aliphatiques, des composés insaturés de type chaîne aromatique et des composés insaturés cycliques aromatiques avec du dioxyde de carbone et de l'hydrogène à l'aide d'un système catalytique comprenant un composé à base de ruthénium et un composé à base de cobalt, dans lequel l'hydroformylation est réalisée à une température de 100 °C à 200 °C à une pression de 1 à 50 MPa.

2. Procédé de production d'alcool selon la revendication 1, dans lequel le système catalytique comprend en outre un sel d'halogénure.

3. Procédé de production d'alcool selon la revendication 1 ou 2, dans lequel le système catalytique comprend en outre un acide.

4. Procédé de production d'alcool selon la revendication 3, dans lequel l'acide est un acide de Bronsted.

5. Procédé de production d'alcool selon la revendication 4, dans lequel l'acide de Bronsted est un acide contenant du phosphore.

6. Procédé de production d'alcool selon l'une quelconque des revendications 1 à 5, dans lequel le composé organique comporte une liaison carbone-carbone insaturée qui ne se situe pas à l'extrémité terminale de sa molécule mais à l'intérieur de la molécule.

7. Procédé de production d'alcool selon l'une quelconque des revendications 1 à 6, dans lequel le composé organique est un composé comportant deux ou plusieurs liaisons carbone-carbone insaturées et un alcool polyvalent est produit à l'aide de ce composé organique.
